(19) Europäisches Patentamt
European Patent Office
Office européen des brevets

(11) **EP 2 399 119 B1**

(12) **EUROPÄISCHE PATENTSCHRIFT**

(45) Veröffentlichungstag und Bekanntmachung des Hinweises auf die Patenterteilung:
**17.04.2013 Patentblatt 2013/16**

(21) Anmeldenummer: **10703910.9**

(22) Anmeldetag: **18.02.2010**

(51) Int Cl.:
***G01N 21/84*** *(2006.01)*

(86) Internationale Anmeldenummer:
**PCT/EP2010/052012**

(87) Internationale Veröffentlichungsnummer:
**WO 2010/094726 (26.08.2010 Gazette 2010/34)**

(54) **Testverfahren und Testvorrichtung zur Untersuchung einer Körperflüssigkeit**

Test device and method for examining a bodily fluid

Procédé de test et dispositif de test destinés à examiner un liquide corporel

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **18.02.2009 EP 09153113**

(43) Veröffentlichungstag der Anmeldung:
**28.12.2011 Patentblatt 2011/52**

(73) Patentinhaber:
• **F. Hoffmann-La Roche AG**
**4070 Basel (CH)**
Benannte Vertragsstaaten:
**AT BE BG CH CY CZ DK EE ES FI FR GB GR HR HU IE IS IT LI LT LU LV MC MK MT NL NO PL PT RO SE SI SK SM TR**
• **Roche Diagnostics GmbH**
**68305 Mannheim (DE)**
Benannte Vertragsstaaten:
**DE**

(72) Erfinder:
• **MILTNER, Karl**
**67227 Frankenthal (DE)**
• **LORENZ, Robert**
**67547 Worms (DE)**
• **PORSCH, Ulrich**
**69469 Weinheim (DE)**
• **KNAPP, Clemens**
**58519 Viernheim (DE)**

(74) Vertreter: **Pfiz, Thomas**
**Patentanwälte Wolf & Lutz**
**Hauptmannsreute 93**
**70193 Stuttgart (DE)**

(56) Entgegenhaltungen:
EP-A1- 1 785 730   WO-A1-99/18426
US-A- 3 526 480   US-A- 5 051 901
US-A- 5 304 468

**Beschreibung**

Die Erfindung betrifft ein Testverfahren zur Untersuchung einer Körperflüssigkeit insbesondere zur Blutzuckerbestimmung bei dem ein Testband vorzugsweise in Form einer Bandkassette in einem Testgerät eingesetzt wird, um eine Vielzahl von auf dem Testband bevorrateten analytischen Testfeldern durch Bandtransport sukzessive bereitzustellen, wobei das jeweils bereitgestellte Testfeld durch einen Benutzer mit der Körperflüssigkeit beaufschlagt wird und mittels einer geräteseitigen Messeinheit unter Erfassung von Messsignalen über die Dauer eines Messintervalls photometrisch abgetastet wird. Die Erfindung betrifft weiter eine entsprechende Testvorrichtung.

Eine gattungsgemäße Testbandvorrichtung ist beispielsweise aus der EP 2177914 (Stand der Technik gemäß Art. 54(3) EPÜ) der Anmelder bekannt. Dort ist eine Bandkassette mit einem Testband beschrieben, auf dem sich neben den analytischen Testfeldern auch Positioniermarken befinden, um für jeden betreffenden Bandabschnitt eine zuverlässige Positionierung in verschiedenen Funktionsstellungen zu gewährleisten.

Aus der DE 199 32 846 A1 ist ein Verfahren zur Erkennung der Fehlpositionierung eines optisch auswertbaren Teststreifens bekannt, das auf dem Vergleich zweier Messwerte aus voneinander in Einschubrichtung des Teststreifens in einem Testgerät beabstandeten Messflecken beruht. Die Verhältnisse bei Teststreifensystemen sind allerdings schon insoweit kaum mit Bandsystemen vergleichbar, als die Teststreifen einzeln in eine Geräteführung eingesetzt werden, während der Bandtransport und die Bandführung auf Seite des Verbrauchsmittels erfolgt.

Die Patentschrift US 5,051,901 offenbart ein Verfahren, wobei eine Körperflüssigkeit mittels eines Testbands untersucht wird, und das Benetzen eines Testfeldes als fehlerhaft erkannt wird, wenn die Differenz zwischen dem Maximum und dem Minimum der zeitabhängigen optischen Dichte einen Schwellwert unterschreitet.

Ausgehend hiervon liegt der Erfindung die Aufgabe zugrunde, die im Stand der Technik vorgeschlagenen Testverfahren und -vorrichtungen weiter zu verbessern und eine erhöhte Sicherheit gegen Fehlbedienung und Fehlmessungen zu gewährleisten.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, aus einer erwarteten Signaländerung eines für das Testergebnis relevanten Messsignals eine Fehleranalyse abzuleiten. Eine auf den Umständen der Probenapplikation beruhende Fehlerdiskriminierung sieht vor" dass die Messsignale bei zwei unterschiedlichen Wellenlängen erfasst werden, dass ein Kontrollwert aus einer Signaldifferenz der wellenlängenunterschiedlichen Messsignale ermittelt wird, wobei die Signaldifferenz durch Benetzen des bereitgestellten Testfelds mit der Körperflüssigkeit erzeugt wird, und dass die Messsignale bei Unterschreiten eines vorgegebenen Schwellenwerts des Kontrollwerts als fehlerhaft verworfen werden.. Auf diese Weise ist es möglich, verfälschende Fremdeinwirkungen auf das Messergebnis weitgehend auszuschließen. Es versteht sich, dass dabei auch mehrere potentielle Fehlerfälle parallel überprüfbar sind. Dadurch lassen sich speziell auch solche Manipulationen erkennen, bei denen ein Benutzer beispielsweise mit dem Finger gegen das Testfeld andrückt, ohne jedoch eine Probe aufzutragen. Speziell beruht die Signaldifferenz der wellenlängenunterschiedlichen Messsignale auf der Benetzung des bereitgestellten Testfelds mit der Körperflüssigkeit, so dass auch bei geringen Analytkonzentrationen eine zuverlässige Fehlererkennung möglich ist.

In diesem Zusammenhang ist es günstig, wenn die wellenlängenunterschiedlichen Messsignale im sichtbaren Wellenlängenbereich und im Infrarotbereich gewonnen werden.

Eine weitere vorteilhafte Ausgestaltung besteht darin, dass der Kontrollwert aus einer Signaldifferenz von am Anfang und Ende eines Messintervalls erfassten Messsignalen ermittelt wird, wobei bei verschwindender Signaldifferenz ein Fehlerfall erkannt wird. Diese Art der Fehlererkennung beruht auf der speziellen Reaktionskinetik eines Analyten auf farbveränderlichen Testfeldern, die sich somit von einer mechanischen Bandmanipulation unterscheiden lässt.

In der Bereitstellungsphase für die Flüssigkeitsapplikation ist es von Vorteil, wenn an dem bereitgestellten Testfeld zyklisch Leerwerte erfasst werden, und wenn der Kontrollwert aus einer Leerwertänderung gegenüber einem anfänglichen Leerwert ermittelt wird, wobei bei einer Leerwertänderung über dem Schwellenwert eine Flüssigkeitsapplikation und unterhalb davon ein Fehlerfall erkannt wird.

Vorteilhafterweise wird bei einer Leerwertänderung bis zu einem vorgegebenen Grenzwert der momentane Leerwert zur Bestimmung eines relativen Messwerts für einen Analyten in der Körperflüssigkeit berücksichtigt. Dadurch lässt sich ein referenzierter Messwert gewinnen, ohne dass eine geringe Änderung der Bezugsgröße zu einem verfälschten Ergebnis führt.

Die vorgenannten Vorteile ergeben sich auch für eine korrespondierende Vorrichtung zur Ausführung des erfindungsgemäßen Verfahrens.

Im Folgenden wird die Erfindung anhand eines in der Zeichnung dargestellten Ausführungsbeispiels näher erläutert. Es zeigen:

Fig. 1 ein analytisches Testbandsystem zur Blutzuckerbestimmung umfassend ein Handgerät und eine Testbandkassette in einer aufgeschnittenen perspektivischen Darstellung;

Fig. 2 eine Ausschnittsvergrößerung der Fig. 1 im Bereich einer Messspitze;

Fig. 3 einen Testbandabschnitt in der Draufsicht;

Fig. 4 ein Messwert-Diagramm in verschiedenen Phasen des Messablaufs;

Fig. 5 ein Messwert-Diagramm in Abhängigkeit der Konzentration eines Analyten für zwei verschiedene Wellenlängen.

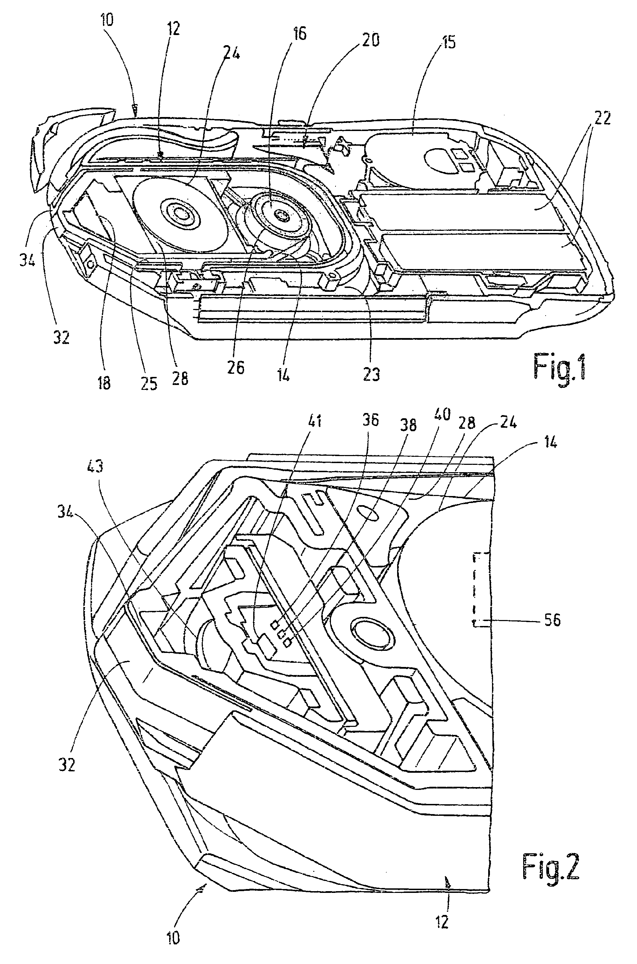

Das in Fig. 1 gezeigte Testbandsystem ermöglicht in einem Handgerät 10 den Einsatz einer Bandkassette 12 mit vorspulbarem Testband 14 als Verbrauchsmittel zur Durchführung von Glukosetests, wobei Funktionsprüfungen in verschiedenen Phasen des Messablaufs vorgenommen werden. Das generelle Geräteprinzip ist in der EP-Anmeldung Nr. 02026242.4 beschrieben, worauf hier Bezug genommen wird.

Das Handgerät 10 besitzt einen Bandantrieb (Motor 15 mit Antriebsspindel 16), eine Messeinheit 18, eine mikroprozessorgestützte Steuereinrichtung 20 und eine Energieversorgung 22. Eine nicht gezeigte Anzeige ermöglicht die Ausgabe von Messergebnissen und Gerätemeldungen für den Benutzer.

Die in ein Aufnahmefach 23 des Geräts 10 einsetzbare Bandkassette 12 umfasst eine Vorratsspule 24 für unverbrauchtes Testband 14 und eine mit dem Antrieb 16 koppelbare Aufwickelspule 26 für verbrauchtes Testband sowie eine Bandführung 25 mit einer Umlenkspitze 34. Die Vorratsspule 24 ist in einer gegenüber der Umgebung abgedichteten Vorratskammer 28 angeordnet.

Das Testband 14 ist abschnittsweise mit Testfeldern 32 versehen, die somit in einer gegebenen Reihenfolge in Bandtransportrichtung gesehen angeordnet sind. Hierbei ist zu berücksichtigen, dass die mit Blut kontaminierten Testfelder 32 auf der Aufwickelspule 26 entsorgt werden und somit ein Rückspulen des Bandes nicht praktikabel ist.

Das jeweils bereitgestellte bzw. aktive Testfeld 32 ist im Bereich der von außen zugänglichen Umlenkspitze 34 vorderseitig mit Probenflüssigkeit, insbesondere Blut oder Gewebeflüssigkeit beaufschlagbar. Der Nachweis des Analyten (Glukose) erfolgt durch rückseitige reflexionsphotometrische Erfassung einer Farbänderung der Testfelder 32 mittels der Messeinheit 18. Zu diesem Zweck sind die Testfelder 32 als Trockenreagenzschicht auf einer transparenten Trägerfolie aufgebracht. Durch entsprechenden Bandvorlauf können die Testfelder 32 sukzessive zum Einsatz gebracht werden. Auf diese Weise lassen sich Vielfachtests für eine Patienten-Selbstkontrolle durchführen, ohne dass Verbrauchsmittel häufig ausgetauscht werden müssten.

Wie in Fig. 2 gezeigt, weist die in die Kassette 12 eingreifende gerätefeste Messeinheit 18 drei Leuchtdioden 36, 38, 40 als Strahlungsquelle sowie eine Photodiode 41 als Detektor für eine reflexionsphotometrische Signalerfassung auf. Eine Optik 43 ermöglicht einen gebündelten Strahlengang unter Abbildung von Leuchtflecken definierter Größe und Intensität auf der Bandrückseite. Die mittlere Leuchtdiode 38 strahlt im sichtbaren (roten) Wellenlängenbereich bei ca. 650 nm, während die äußeren Leuchtdioden 36, 40 im Infrarot bei 875 nm arbeiten. Das auf dem Teststreifen 48 rückwärts gestreute Licht wird mit der Photodiode 41 in einem gegebenen Zeittakt erfasst.

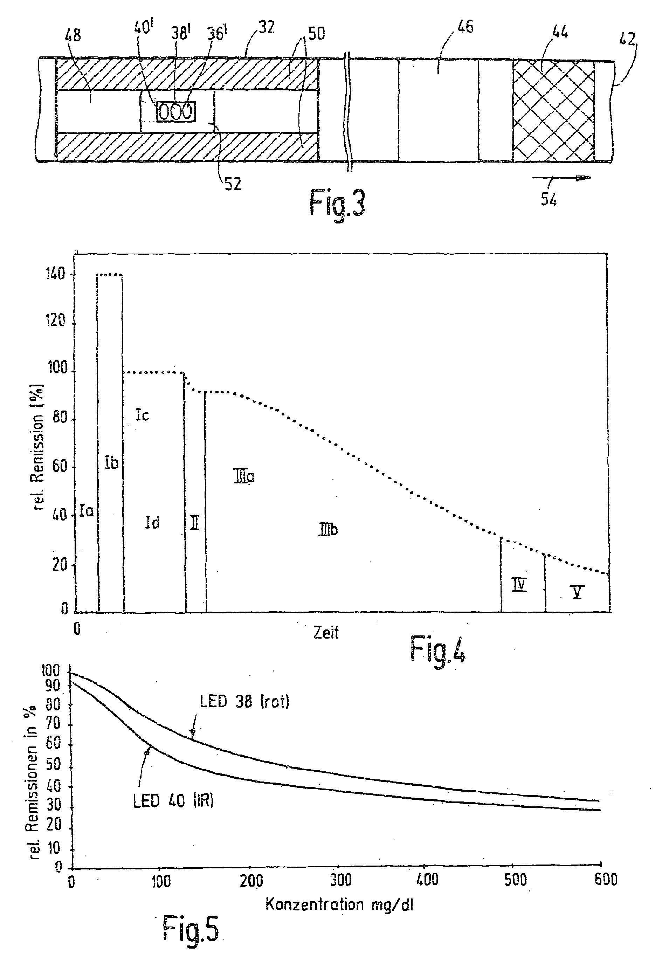

Wie in Fig. 3 veranschaulicht, befinden sich die voneinander beabstandeten Testfelder 32 einzeln auf jeweils einem zugeordneten Bandabschnitt 42, der zusätzlich mit weiteren Prüf- bzw. Kontrollfeldern in Form eines Schwarzfelds 44 und eines Weißfelds 46 versehen ist. Das Testfeld 32 weist einen durch die Testchemieschicht gebildeten zentralen Teststreifen 48 auf, der von zwei hydrophoben Randstreifen 50 seitlich begrenzt ist. Die auf dem Testfeld 32 vorderseitig applizierte Probenflüssigkeit benetzt den Teststreifen 48 in Form eines Probenflecks 52, der durch die Leuchtflecken 36', 38', 40' der Leuchtdioden 36,38,40 in der Messposition an der Umlenkspitze 34 von der transparenten Bandrückseite her abgetastet wird. Aufgrund des nur in einer Richtung (Pfeil 54) möglichen Bandtransports werden allerdings zunächst die Prüffelder 44, 46 vor der eigentlichen Messung erfasst, wie nachstehend näher erläutert.

In der Standby-Position befindet sich das Weißfeld 46 des noch unbenutzten Bandabschnitts 42 an der Umlenkspitze 34 vor der Messeinheit 18. Das in weißer Farbe auf das Trägerband 34 aufgedruckte Weißfeld 46 ist so bemessen, dass das von der Messeinheit 18 erfasste Messfenster vollständig überdeckt wird. In gleicher Weise kann auch ein vorgeordnetes Schwarzfeld 44 vor Einnahme der Standby-Position zur Vermessung positioniert werden.

Wie aus Fig. 4 ersichtlich, ist der Messzyklus für jeden Bandabschnitt 42 in verschiedene Phasen unterteilt. In der Phase Ia wird das Schwarzfeld 44 zur Verschmutzungserkennung und ggf. zur geräteseitigen Eigenkorrektur abgetastet, wie es nachstehend noch näher erläutert wird. In Phase Ib erfolgt die Vermessung des Weißfelds 46 zur Bandqualitätsüberprüfung und ggf. zur Eigenkorrektur. Phase Ic sieht die Gewinnung eines so genannten Trockenleerwerts TLW an dem noch unbenutzten Testfeld 32 vor. Sodann ergeht eine Aufforderung zum Blutauftrag an den Benutzer (Id). Die Bereitstellungsphase ist damit abgeschlossen.

In Phase II erfolgt eine Benetzungserkennung an dem Testfeld 32 mittels der IR-Leuchtdioden 36, 40. Bei der Benetzung des Teststreifens 48 findet ein Einbruch der Signalintensität statt.

Anschließend wird die Kinetik des analytspezifischen Messsignals anhand der Farbveränderung des Teststreifens 48 in den Phasen III und IV im Messtakt von z.B. 0,2 s verfolgt. Die Endphase IIIb der Kinetikverfolgung ergibt sich bei Erreichen einer Abbruchschwelle der sich in Abhängigkeit von der chemischen Reaktionsgeschwindigkeit abschwächenden Signaländerung. Sodann wird in Phase IV eine Zweifachmessung mittels LED 38 vorgenommen, um einen gemittelten Endwert EW zu ermitteln. Die Bestimmung der Glukosekonzentration erfolgt dann in relativer Remission, indem der Quotient aus diesem Endwert EW und dem Trockenleerwert TLW gebildet wird (allgemein errechnet sich die relative Remission aus dem Verhältnis des aktuellen Messwerts zum Trockenleerwert). Phase V sieht noch eine Homogenitätsmessung des Probenflecks 52 zur Unterdosiererkennung vor, die auf dem quantitativen Signalvergleich der beiden IR-LEDs 36, 40 beruht. Im Display des Geräts 10 wird schließlich die Glukosekonzentration für den Benutzer angezeigt.

Außer der eigentlichen Messung zur Bestimmung der Glukosekonzentration werden die oben angesprochenen Funktionen bzw. "Fail-Safes" folgendermaßen realisiert:

　　Die Verschmutzungserkennung erfolgt mit LED 38 nach dem Einlegen der Kassette 12 und im Anschluss an jede Glukosemessung über eine Messung des Signaloffsets auf dem Schwarzfeld 44. Dieser Offset wird von dem gesamten Messumfeld bei eingeschalteten LEDs erzeugt, ohne dass ein Test beteiligt ist. Er geht damit als additive Größe bei der Ermittlung des Messwerts ein. Aufgrund von Verschmutzungen oder sonstigen Optikveränderungen im Strahlengang beispielsweise durch Fremdkörper, Staub und Kratzer wird ausgesendetes Licht teilweise reflektiert und zum Detektor 41 geleitet. Das Schwarzfeld 44 dient bei der Offseterkennung als Ersatz für einen schwarzen Hohlraum, der kein Licht zurückwirft. Grundsätzlich wäre es aber auch möglich, durch das transparente Trägerband hindurch in den dunklen Geräteinnenraum hinein zu messen.

Der erfasste Signaloffset wird mit einem im Gerät 10 gespeicherten Grenzwert verglichen, der bei der Produktherstellung als chargenmittelwert ermittelt wurde. Wird der hinterlegte Grenzwert überschritten, so wird eine Fehlermeldung ausgelöst.

Um die Qualität der als Einwegartikel ggf. nach längerer Lagerzeit eingesetzten Bandkassette 12 zu überprüfen, wird zumindest an dem ersten Weißfeld 46 auf dem Testband 14 ein Referenzwert WF erfasst. Anschließend wird eine potentielle Schädigung der Teststreifenchemie beispielsweise aufgrund von Umwelteinflüssen durch eine entsprechende Veränderung des Trockenleerwerts TLW des ersten Testfelds 32 nachgewiesen. Dazu wird nicht der absolute Remissionswert herangezogen, sondern der auf den Referenzwert WF bezogene relative Remissionswert.

Ein entsprechender Chargenkontrollwert CC wird im Zuge einer der chargenweisen Herstellung von Testbändern 14 durch Vermessen von Testfeldern 32 und Weißfeldern 46 auf dem Testbandmaterial bestimmt. Die Bandherstellung erfolgt in einem Rolle-zu-Rolle-Prozess, der eine solche Kontrollwertzuordnung zu einer weitgehend einheitlichen Beschichtung erlaubt. Der Chargenkontrollwert wird in einem RFID-Chip 56 auf der Kassette 12 gespeichert und durch die Geräteelektronik 20 ausgelesen und verarbeitet. Der RFID-Chip 56 ist außenseitig auf der Kassette 12 aufgebracht und in der aufgeschnittenen Darstellung nach Fig. 2 nur symbolisch gezeigt.

Die Testfeld-Qualitätsprüfung fällt negativ aus, wenn folgende Bedingung erfüllt ist:

$$TLW_1 / WF_1 < CC - \Delta C \quad (1)$$

wobei $\Delta C$ ein Toleranzwert ist und der Index 1 sich auf den ersten Testbandabschnitt 42 bezieht. In diesem Fall wird eine entsprechende Fehlermeldung ausgegeben und die Kassette 12 ggf. verworfen.

Bei positivem Ergebnis ist es auch möglich, für die nachfolgenden Tests eine Qualitätsprüfung in engeren Schranken durchzuführen. Hierzu wird der relative Remissionswert $Cn-1$ des aktuell benutzten Testfelds in einem Gerätespeicher abgespeichert und entsprechend der obenstehenden Gleichung (1) anstelle des Chargenkontrollwerts CC eingesetzt. Eine nachfolgende Qualitätsprüfung eines nächsten Testfelds n fällt also negativ aus, wenn:

$$TLW_n / WF_n < C_{n-1} - \Delta C \quad (2).$$

Generell wird durch eine herstellerseitige Kalibrierung der Geräte 10 sichergestellt, dass relevante Messwerte nur im spezifizierten Messbereich aller optoelektronischen Komponenten erzeugt werden können. Hierbei erfolgt eine Kalibrierung der elektrischen Parameter der drei LEDs 36, 38, 40 und der optischen Kenngrößen.

Prinzipiell ist auch ein Eigenkorrekturverfahren bzw. eine geräteseitige Kalibrierung möglich, um den spezifischen Einfluss des Signaloffsets und schwankender Absolutmesswerte auf die Messwertbestimmung zu minimieren. Herstellungsbedingt variiert der optische Offset von Kassette zu Kassette. Hinzu kommt, dass aufgrund der Trennung von geräteseitiger Optik und kassettenseitiger Bandführung ein mit Toleranzen behafteter Abstandsanteil auftritt.

Für die Referenzmessung dienen wiederum die Schwarz- und Weißfelder 44, 46, die sich auf dem Testband 14 vor jedem Testfeld 32 befinden. Bereits in der Produktion werden diese Felder vermessen und mit einem Chargenmittelwert versehen. Diese Werte werden als Referenzwert auf dem RFID-Chip 56 abgelegt.

Der nach dem Einlegen einer Kassette 12 an dem ersten Schwarzfeld 44 gemessene Schwarzfeldwert wird überprüft, ob er mit einer vorgegebenen Toleranz am herstellerseitigen Chargenmittelwert für den optischen Offset liegt. Ist dies der Fall, wird der Chargenmittelwert beibehalten. Weicht der gemessene Schwarzfeldwert von diesem Toleranzbereich ab, so wird die Differenz zu dem Chargenmittelwert bestimmt und dem optischen Offset zugerechnet. Der optische Offset wird bei den nachfolgend an den Testfeldern 32 gewonnenen Brutto-Messsignalen subtrahiert.

Die Korrektur des optischen Offsets wird jedoch nur bis zu einem festgelegten Grenzwert durchgeführt. Bei einer Überschreitung dieser Grenze wird wie oben beschrieben eine Fehlermeldung ausgelöst. Vor jedem nachfolgenden Test wird die Schwarzfeldmessung nur zur Verschmutzungserkennung verwendet.

Bei der Weißfeldkalibrierung wird der individuelle Empfindlichkeitswert der Kassette bestimmt, indem der am Weißfeld 46 erfasste Messwert mit dem auf dem RFID-Chip 56 hinterlegten Chargenmittelwert in absoluter Remission verglichen wird. Liegt der gemessene WeißfeldweA $m_K$ innerhalb eines Toleranzbereichs am Chargenmittelwert $m_W$, so wird für eine nachfolgende Skalierung des Offset-korrigierten Brutto-Messsignals der Chargenmittelwert $m_W$, herangezogen und ansonsten die individuelle Kassettenempfindlichkeit $m_K$. Bei der Überschreitung eines Grenzwertes der Abweichung wird jedoch eine Fehlermeldung ausgegeben.

Um eine ungewollte Messwerterzeugung durch Fehlbedienung weitgehend auszuschließen, kann ein Kontrollwert aus einer zeitlichen und/oder wellenlängenabhängigen Änderung der Messsignale ermittelt werden, wobei die Messsignale dann in Abhängigkeit von einem Schwellenwer des Kontrollwerts als gültig weiter verarbeitet oder als fehlerhaft verworfen werden.

Ein erster solcher Fehlerfall kann darin bestehen, dass aufgrund der Abstandsabhängigkeit des Messprinzips durch Andrücken gegen die Umlenkspitze 34 ein Messstart und ein artifizielles Messergebnis erzeugt werden könnte, ohne dass eine Probe appliziert wurde. Um dies ausschließen zu können, werden Messsignale an dem Testfeld 32 bei zwei unterschiedlichen Wellenlängen erfasst, wobei der Kontrollwert aus einer Signaldifferenz der wellenlängenunterschiedlichen Messsignale ermittelt wird.

Wie aus Fig. 5 ersichtlich, ergeben sich bei einer Probenvermessung mit unterschiedlichen Wellenlängen unterschiedliche Werte der relativen Remission über den gesamten Bereich der zu untersuchenden Analyt- bzw. Glukosekonzentration. Dieser Signalunterschied entsteht durch Befeuchten des Testfelds 32 mit der Körperflüssigkeit (deshalb ist auch bei Probenkonzentration 0 bereits ein Unterschied zu finden) und verstärkt sich, wenn das Testchemiesystem verstärkt Reaktionsfarbe bildet. Wird also nur durch Andrücken ein Messsignal erzeugt, kann der typische Unterschied in den beiden wellenlängenunterschiedlichen LEDs 38, 40 durch das fehlende Befeuchten und die fehlende Reaktionsfarbe nicht beobachtet werden, wodurch ein Fehlerfall erkannt wird. Der vorgegebene Schwellenwert des Signalunterschieds kann beispielsweise bei 3% relativer Remission liegen.

Ein weiteres Szenario einer ungewollten Messwerterzeugung besteht darin, dass die fälschliche Erkennung eines Blutauftrags durch eine Bandverschiebung provoziert wird. Wird durch eine Benutzermanipulation ein dunkler Randstreifen 50 des Testfelds 32 in den Strahlengang der Messeinheit 18 verschoben, können hohe Messwerte erzeugt werden, ohne dass Probe aufgetragen wurde.

Typische Reaktionskinetiken von Blutproben auf einem Testfeld 32 zeigen jedoch oberhalb von ca. 100 mg/dl Glukosekonzentration einen Signalhub von ca. 10%, wie er als Differenz der ersten und letzten Kinetikmessung in Phase III (Fig. 4) ermittelt wird. Wird hingegen das Testband in Phase II wie oben beschrieben bloß verschoben, erfolgt ein schlagartiges Verdunkeln und danach ein konstantes Signal - es wird also in Phase III keine veränderliche Reaktionskinetik und kein nennenswerter Signalhub beobachtet. Somit ist eine Fehlererkennung dadurch möglich, dass der Kontrollwert aus einer Signaldifferenz von am Anfang und Ende eines Messintervalls erfassten Messsignalen ermittelt wird, und dass bei einer Signaldifferenz nahe Null ein Fehlerfall erkannt wird.

Befindet sich ein Testfeld 32 vor der Optik 43 im Zustand der Probenauftragserkennung (Phase II in Fig. 4), so interpretiert die Steuereinrichtung 20 des Geräts 10 eine Signaländerung um ein festgelegtes Maß als Probenauftrag und beginnt mit der Auswertung. Sowohl hohe Luftfeuchtigkeit als auch Sonneneinstrahlung können unter ungünstigen Umständen ohne Probenauftrag bereits zu einer solchen Signal änderung und damit zu einem Messstart führen.

Um dies zu verhindern, wird der zeitliche Verlauf der Leersignaländerung im Zustand der Probenerwartung geprüft. Während das Auftragen einer Blutprobe bereits innerhalb einer halben Sekunde einen Remissionsabfall von mehreren Prozent erzeugt, wird ein solcher Abfall bei Einwirkung von Sonnenlicht oder Luftfeuchte erst über einen Zeitraum von mehr als 20 Sekunden erreicht. Demzufolge ist es möglich, dass an dem für die Probenapplikation bereitgestellten Testfeld zyklisch Leerwerte erfasst werden, und dass der Kontrollwert aus einer Leerwertänderung gegenüber einem anfänglichen Leerwert ermittelt wird, wobei bei einer Leerwertänderung über einem vorgegebenen Schwellenwert (von beispielsweise ca. 5%) eine Flüssigkeitsapplikation und unterhalb davon ggf. nach einer gegebenen Wartezeit ein Fehlerfall erkannt wird.

Ein weiteres Messproblem kann darin bestehen, das sich der Trockenleerwert eines bereitgestellten, aber noch unbenutzten Testfelds 32 beispielsweise durch Licht- oder Feuchtigkeitseinfluss verändert und damit als Bezugsgröße für die Bestimmung der relativen Remission zu einer Verfälschung führt. Der Messwert des unbenutzten Testfelds kann daher im Zustand der Probenerwartung zyklisch überprüft und entweder nachgeführt werden, um somit Messwertverfälschungen zu verhindern, oder ab einem bestimmten Grenzwert beispielsweise von mehr als 0,5%/s relativer Remissionsänderung die Messung mit einer Fehlermeldung abzubrechen.

**Patentansprüche**

1. Testverfahren zur Untersuchung einer Körperflüssigkeit insbesondere zur Blutzuckerbestimmung bei dem ein Testband (14) vorzugsweise in Form einer Bandkassette (12) in einem Testgerät (10) eingesetzt wird, um eine Vielzahl von auf dem Testband (14) bevorrateten analytischen Testfeldern (32) durch Bandtransport sukzessive bereitzustellen, wobei das jeweils bereitgestellte Testfeld (32) durch einen Benutzer mit der Körperflüssigkeit (52) beaufschlagt wird und mittels einer geräteseitigen Messeinheit (18) unter Erfassung von Messsignalen über die Dauer eines Messintervalls photometrisch abgetastet wird, **dadurch gekennzeichnet, dass** die Messsignale bei zwei unterschiedlichen Wellenlängen erfasst werden, dass ein Kontrollwert aus einer Signaldifferenz der wellenlängenunterschiedlichen Messsignale ermittelt wird, wobei die Signaldifferenz durch Benetzen des bereitgestellten Testfelds (32) mit der Körperflüssigkeit erzeugt wird, und dass die Messsignale bei Unterschreiten eines vorgegebenen Schwellenwerts des Kontrollwerts als fehlerhaft verworfen werden.

2. Testverfahren nach Anspruch 1, **dadurch gekennzeichnet, dass** die wellenlängenunterschiedlichen Messsignale im sichtbaren Wellenlängenbereich und im Infrarotbereich gewonnen werden.

3. Testverfahren nach Anspruch 1, **dadurch gekennzeichnet, dass** der Kontrollwert aus einer Signaldifferenz von am Anfang und Ende eines Messintervalls erfassten Messsignalen ermittelt wird.

4. Testverfahren nach einem der Ansprüche 1 bis 3, **dadurch gekennzeichnet, dass** an dem für die Flüssigkeitsapplikation bereitgestellten Testfeld (32) zyklisch Leerwerte erfasst werden, und dass der Kontrollwert aus einer Leerwertänderung gegenüber einem anfänglichen Leerwert ermittelt wird, wobei bei einer Leerwertänderung über dem Schwellenwert das Vorhandensein einer Körperflüssigkeit erkannt wird.

5. Testverfahren nach Anspruch 4, **dadurch gekennzeichnet, dass** bei einer Leerwertänderung bis zu einem vorgegebenen Grenzwert der momentane Leerwert zur Bestimmung eines relativen Messwerts für einen Analyten in der Körperflüssigkeit berücksichtigt wird.

6. Testvorrichtung zur Untersuchung einer Körperflüssigkeit insbesondere zur Blutzuckerbestimmung mit einem Testgerät (10) und einem darin vorzugsweise in Form einer Bandkassette (12) eingesetzten Testband (14), welches eine Vielzahl von analytischen Testfeldern (32) jeweils in einem zugeordneten Bandabschnitt (42) aufweist, wobei die Testfelder (32) durch Bandtransport sukzessive zum Auftragen von Körperflüssigkeit (52) bereitstellbar und mittels einer geräteseitigen Messeinheit (18) unter Erfassung von Messsignalen über die Dauer eines Messintervalls abtastbar sind, **dadurch gekennzeichnet**, die Messeinheit (18) die Messsignale bei zwei unterschiedlichen Wellenlängen erfasst, und dass das Testgerät (10) eine Steuereinrichtung (20) aufweist, die dazu ausgebildet ist, einen Kontrollwert aus einer durch Benetzen des bereitgestellten Testfelds (32) mit der Körperflüssigkeit erzeugten Signaldifferenz der wellenlängenunterschiedlichen Messsignale zu ermitteln und die Messsignale nach Maßgabe des Kontrollwerts als gültig zu verarbeiten oder als fehlerhaft zu verwerfen.

**Claims**

1. Test method for analysing a body fluid in particular for blood sugar determination in which a test tape (14) preferably in the form of a tape cassette (12) is used in a test device (10) in order to successively provide a plurality of analytical test fields (32) stored on the test tape (14) by means of tape transport, wherein the body fluid (52) is applied by a user to the test field (32) provided at a time and the said test field is photometrically scanned using a measuring unit (18) of the device to detect measurement signals over the duration of a measurement interval, **characterized in that** the measurement signals are detected at two different wavelengths, that a control value is determined from a signal difference of the measurement signals at different wavelengths, wherein the signal difference is generated by wetting the provided test field (32) with the body fluid, and that the measurement signals are discarded as erroneous when they fall below a preset threshold of the control value.

2. Test method according to claim 1, **characterized in that** the measurement signals measured at different wavelengths are obtained in the visible wavelength range and in the infrared range.

3. Test method according to claim 1, **characterized in that** the control value is determined from a signal difference of measurement signals detected at the beginning and end of a measurement interval.

4. Test method according to one of the claims 1 to 3, **characterized in that** blank values are detected cyclically on the test field (32) that is provided for liquid application, and that the control value is determined from a change in the blank value compared to an initial blank value, wherein the presence of a body fluid is sensed when the change in blank value is above the threshold value.

5. Test method according to claim 4, **characterized in that** in the case of a change in the blank value up to a preset limit value the current blank value is accounted for determining a relative measurement value for an analyte in the body fluid.

6. Test device for analysing a body fluid in particular for blood sugar determination comprising a test device (10) and a test tape (14) used therein preferably in the form of a tape cassette (12) which has a plurality of analytical test fields (32) each in a dedicated section of tape (42) wherein the test fields (32) can be successively provided for application of body fluid (52) by means of tape transport and can be scanned using a measuring unit (18) of the device to detect measurement signals over the duration of a measurement interval, **characterized in that** the measuring unit (18) detects the measurement signals at two different wavelengths, and that the test device (10) has a control device (20) which is designed to determine a control value from a signal difference of the measurement signals at different wavelengths, said signal difference being generated by wetting the provided test field (32) with the body fluid, wherein the control device (20) is designed to process the measurement signals as valid or to discard the measurement signals as erroneous depending on the control value.

## Revendications

1. Procédé de test destiné à analyser un liquide corporel, en particulier destiné à déterminer la glycémie, dans lequel une bande de test (14) de préférence en forme de cassette à bande (12) est placée dans un appareil de test (10), afin de mettre à disposition de manière successive par le biais du transport de la bande une pluralité de champs de test analytiques (32) stockés sur la bande de test (14), le champ de test (32) respectivement mis à disposition étant exposé au liquide corporel (52) et analysé de manière photométrique au moyen d'une unité de mesure (18) côté appareil lors de la détection de signaux de mesure relatifs à la durée d'un intervalle de mesure, **caractérisé en ce que** les signaux de mesure sont détectés en cas de deux longueurs d'onde différentes, **en ce qu'**une valeur de contrôle est déterminée à partir d'une différence de signal des signaux de mesure ayant des longueurs d'onde différentes, la différence de signal étant générée en mouillant le champ de test (32) mis à disposition avec le liquide corporel, et **en ce que** les signaux de mesure sont rejetés comme étant erronés lorsqu'ils ont une valeur inférieure à une valeur seuil prédéfinie de la valeur de contrôle.

2. Procédé de test selon la revendication 1, **caractérisé en ce que** les signaux de mesure ayant des longueurs d'onde différentes sont obtenus dans le domaine des longueurs d'onde visibles et dans le domaine infrarouge.

3. Procédé de test selon la revendication 1, **caractérisé en ce que** la valeur de contrôle est déterminée à partir d'une différence de signal des signaux de mesure détectés au début et à la fin d'un intervalle de mesure.

4. Procédé de test selon l'une des revendications 1 à 3, **caractérisé en ce que** sur le champ de test (32) mis à disposition pour l'application de liquide des valeurs vierges sont détectées de manière cyclique, et **en ce que** la valeur de contrôle est déterminée à partir d'une modification des valeurs vierges par rapport à une valeur vierge initiale, la présence d'un liquide corporel étant reconnue lorsque la modification de la valeur vierge est supérieure à la valeur seuil.

5. Procédé de test selon la revendication 4, **caractérisé en ce que** lors d'une modification des valeurs vierges jusqu'à une valeur limite prédéfinie, la valeur vierge momentanée est prise en compte afin de déterminer une valeur de mesure relative pour un analyte dans le liquide corporel.

6. Dispositif de test destiné à analyser un liquide corporel, en particulier destiné à déterminer la glycémie avec un appareil de test (10) et une bande de test (14) de préférence en forme de cassette à bande (12) placée dedans, laquelle bande présente une pluralité de champs de test analytiques (32) respectivement dans une partie de bande associée (42), les champs de test (32) pouvant être mis à disposition de manière successive par le biais du transport de la bande pour l'application du liquide corporel (52) et analysés au moyen d'une unité de mesure (18) côté appareil lors de la détection de signaux de mesure relatifs à la durée d'un intervalle de mesure, **caractérisé en ce que** l'unité de mesure (18) détecte les signaux de mesure en cas de deux longueurs d'onde différentes, et **en ce que** l'appareil de test (10) présente un système de commande (20), qui est configuré pour déterminer une valeur de contrôle à partir d'une différence de signal des signaux de mesure ayant des longueurs d'onde différentes générée lorsque le champ de test (32) mis à disposition est mouillé avec le liquide corporel et pour traiter les signaux de mesure comme étant valides ou les rejeter comme étant erronés conformément à la valeur de contrôle.

Fig.1

Fig.2

Fig.3

Fig.4

Fig.5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2177914 A
- DE 19932846 A1
- US 5051901 A
- EP 02026242 A